# EUROPEAN PATENT APPLICATION

(11) **EP 4 454 644 A1**
(43) Date of publication of application: **30.10.2024**
(21) Application number: 22911260.2
(22) Date of filing: 21.12.2022
(51) Int. Cl.: A61K 31/122, A61K 47/36, A61K 47/38, A61P 9/00, A61P 17/18, A61P 39/06, A61P 43/00, C07C 46/10, C07C 50/06

(54) **METHOD FOR STORING REDUCED COENZYME Q10**

(30) Priority: 24.12.2021 JP 2021210438; 26.09.2022 JP 2022152257
(71) Applicant: Kaneka Corporation, Osaka-shi, Osaka 530-8288 (JP)
(72) Inventor: YOKOCHI Yuichi, Takasago-shi, Hyogo 676-8688 (JP); KITAMURA Shiro, Osaka-shi, Osaka 530-8288 (JP); FUKUYAMA Yuka, Takasago-shi, Hyogo 676-8688 (JP)
(74) Representative: Hoffmann Eitle
(86) International application number: PCT/JP2022/047023
(87) International publication number: WO 2023/120558

(57) **Abstract**

The present description discloses a method for storing reduced coenzyme Q10 (QH), which is capable of controlling oxidation. A first embodiment of the present invention relates to a method for storing QH, including storing a composition containing QH and having a water activity at 25°C of 0.45 or less. A second embodiment of the present invention relates to a method for controlling oxidation of QH, including storing a composition containing QH and having a water activity at 25°C of 0.45 or less. A third embodiment of the present invention relates to a composition containing QH and having a water activity at 25°C of 0.45 or less.

## Description

### Technical Field

One or more embodiments of the present invention relate to a method for storing reduced coenzyme Q10, a method for controlling oxidation of reduced coenzyme Q10, and a composition comprising reduced coenzyme Q10.

### Background Art

Coenzyme Q is an essential component widely distributed in living organisms from bacteria to mammals, and is known as a member of mitochondrial electron transfer system in cells in the living organisms. The major component in humans is coenzyme Q10 which is one having 10 repeating structures in the side chain of coenzyme Q, and generally, about 40% to 90% thereof is present in the living body as the reduced form. The physiological activity of coenzyme Q includes activation of energy production by mitochondrial activation, activation of cardiac function, stabilization of cell membranes, and protection of cells by antioxidant activity.

While coenzyme Q10 currently produced and sold is, in large part, oxidized coenzyme Q10, reduced coenzyme Q10 (hereinafter sometimes referred to as "QH") which exhibits higher oral absorbability than that of oxidized coenzyme Q10 has also been commercially available and has come to be used in recent years.

Reduced coenzyme Q10 is easily oxidized, which leads to a problem that the storage cost is high and the scope of application of commercial products is restricted.

A common method for obtaining reduced coenzyme Q10 has already been disclosed (Patent Literature 1). Patent Literature 2 reports that crystal polymorphism is found in reduced coenzyme Q10. Patent Literature 2 reports that a newly appearing crystal form (wherein this crystal is hereinafter referred to as a "reduced coenzyme Q10 Form II crystal", "QH Form II crystal" or "Form II crystal") is much more stable than the conventional reduced coenzyme Q10 (wherein this crystal is hereinafter referred to as a "reduced coenzyme Q10 Form I crystal", "QH Form I crystal" or "Form I crystal") and also has other excellent physical properties.

Patent Literature 3 describes, as a method for producing a reduced coenzyme Q10, a method for producing a reduced CoQ10-CD clathrate by mixing a coenzyme Q10-cyclodextrin clathrate (CoQ10-CD clathrate) and an antioxidant, and then storing the mixture under an atmosphere having a temperature of 10°C to 100°C and a humidity of 0% to 100%. As described in Patent Literature 3, the production rate of reduced CoQ10 was low when a mixture of CoQ10 which was not a CD clathrate and an antioxidant was stored under a condition having a temperature of 60°C and a humidity of 75%, whereas reduced CoQ10 was produced in a large amount when a mixture of a CoQ10-CD clathrate and an antioxidant was stored under the same condition.

Patent Literature 4, Patent Literature 5, and Patent Literature 6 describe, as reduced coenzyme Q10 having high oxidation stability and high *in vivo* absorbability, a particulate composition in which an oily component containing reduced coenzyme Q10 or an oily component containing reduced coenzyme Q10 and a lipophilic antioxidant forms a domain and is poly-dispersed in a matrix containing a water-soluble excipient or a matrix containing a water-soluble excipient and water-soluble ascorbic acids, and describe a method for stabilizing the particulate composition by placing the particulate composition in an ambient environment having a relative humidity of 90% or less. Examples of the water-soluble excipient described include gum arabic and gelatin.

Patent Literature 7 describes, as a formulation for preventing reduced coenzyme Q10 from oxidation, a reduced coenzyme Q10 solid formulation in which a solid composition containing reduced coenzyme Q10 is coated with at least one coating material selected from the group consisting of an oil-soluble coating material and a water-soluble coating material, and describes a method in which the formulation is placed in an environment adjusted to a relative humidity of 75% or less. As the oil-soluble coating material, shellac and zein are exemplified. As the water-soluble coating material, gelatin, sugar, gum arabic, pullulan, a cellulose derivative and a yeast cell wall are exemplified.

Patent Literature 8 describes a method for storing reduced coenzyme Q10, comprising producing or obtaining a capsule containing reduced coenzyme Q10 and controlling the surrounding environment of the capsule to a relative humidity of 0% to 60%. Examples of the capsule material described include gelatin.

A protein coating film such as gelatin is known to have increased oxygen permeability and decreased gas barrier properties under a high humidity condition. For example, Non-Patent Literature 1 describes that a 20% increase in relative humidity causes a 10-fold increase in the oxygen permeability of a gelatin film containing no glycerin. Non-Patent Literature 2 describes that increasing water activity or relative humidity tends to increase from 10 to 10⁵ times the oxygen permeability, and that, for example, oxygen permeability of a collagen film is 6.6 × 10⁻¹⁹ g m⁻¹ s⁻¹ Pa⁻¹ at a water activity of 0, while it is 13.68 × 10⁻¹⁵ g m⁻¹ s⁻¹ Pa⁻¹ at a water activity of 0.93. Non-Patent Literature 3 describes that increasing plasticizer amount, temperature, and RH generally increases oxygen and water vapor permeability of a protein film.

All of Patent Literature 4 to 8 disclose a technique for increasing oxidation stability by coating QH with a coating film of a gas barrier material such as gelatin, gum arabic, or shellac. Patent Literature 4 to 8 describe stabilization of QH in storage of a QH formulation coated with a gas barrier coating film such as gelatin at a relative humidity equal to or less than a predetermined value. In view of the known information that oxygen permeability of a gas barrier coating film composed of protein such as gelatin is increased under a high humidity condition as described above, it can be understood that, in Patent Literature 4 to 8, oxygen permeability of the gas barrier coating film is reduced and oxidation of QH is controlled by controlling the relative humidity to a value equal to or less than a predetermined value when a QH formulation coated with a gas barrier coating film is in storage.

Patent Literature 9 describes a co-crystal containing reduced coenzyme Q10 and a compound such as 3,4-dihydroxybenzoic acid, which has been found as an additional form of reduced coenzyme Q10. Patent Literature 10 describes formation of a co-crystal of reduced coenzyme Q10 and nicotinamide. Although reduced coenzyme Q10 may have increased oxidation stability due to co-crystallization of reduced coenzyme Q10 and one or more other compounds, even such co-crystallization cannot completely prevent reduced coenzyme Q10 from being oxidized.

### Citation List

### Patent Literature

Patent Literature 1: JP Patent Publication No. H10-109933 A
Patent Literature 2: WO2012/176842
Patent Literature 3: JP Patent Publication No. 2010-126492 A
Patent Literature 4: WO2007/148798
Patent Literature 5: WO2008/129980
Patent Literature 6: JP Patent Publication No. 2009-149584 A
Patent Literature 7: WO2006/075502
Patent Literature 8: JP Patent Publication No. 2006-206583 A
Patent Literature 9: WO2019/162429
Patent Literature 10: Chinese Patent Publication No. CN113024362A

### Non-Patent Literature

Non-Patent Literature 1: Soft Gelatin Capsules II: Oxygen Permeability Study of Capsule Shells, Hom FS, Veresh SA, and Ebert WR, Journal of Pharmaceutical Sciences, 1975, 64(5):851-857
Non-Patent Literature 2: Handbook of Encapsulation and Controlled Release, CRC Press, 2016, Edited by Munmaya Mishra, p.818
Non-Patent Literature 3: Protein-Based Films and Coatings (Book), CRC press, 2002, edited by Aristippos Gennadios, CHAPTER 1 (by John M Korochta), p.12

### Summary of Invention

### Technical Problem

Patent Literature 3 to 8 disclose a QH product that prevents oxidation of reduced coenzyme Q10 (QH) and can be stably stored. However, all of Patent Literature 3 to 8 require QH to be formed into a formulation, for example, a clathrate, a coated body, a particulate composition, or a capsule, with a specified component, and therefore applications of QH are limited. In addition, in a case in which QH is not formed into a composite or a formulation with a specific component as in Patent Literature 3 to 8, it is expected that the lower humidity in a storage environment leads to the higher the percentage of oxygen in the air, promoting the QH oxidation.

One or more embodiments of the present invention provide a method for storing QH, a method for controlling oxidation of the QH, and a composition containing the QH, which are capable of controlling oxidation of QH.

### Solution to Problem

The present inventors have unexpectedly found that QH has higher oxidation stability in a composition having a low water activity of 0.45 or less, and have completed the following respective aspects of the present invention.
(1) A method for storing reduced coenzyme Q10, including
   storing a composition containing reduced coenzyme Q10 and having a water activity at 25°C of 0.45 or less.
(2) The method according to (1), wherein the composition is a mixed composition in which the reduced coenzyme Q10 and one or more other components are mixed.
(3) The method according to (1), wherein the composition is a multiphase composition including a first phase containing the reduced coenzyme Q10 and a second phase containing one or more other components, the first and second phases being in contact with each other.
(4) A method for controlling oxidation of reduced coenzyme Q10, including
   storing a composition containing reduced coenzyme Q10 and having a water activity at 25°C of 0.45 or less.
(5) The method according to (4), wherein the composition is a mixed composition in which the reduced coenzyme Q10 and one or more other components are mixed.
(6) The method according to (4), wherein the composition is a multiphase composition including a first phase containing the reduced coenzyme Q10 and a second phase containing one or more other components, the first and second phases being in contact with each other.
(7) A composition containing reduced coenzyme Q10 and having a water activity at 25°C of 0.45 or less.
(8) The composition according to (7), wherein the composition is a mixed composition in which the reduced coenzyme Q10 and one or more other components are mixed.
(9) The composition according to (7), wherein the composition is a multiphase composition including a first phase containing the reduced coenzyme Q10 and a second phase containing one or more other components, the first and second phases being in contact with each other.
(10) The method according to any one of (1) to (3), wherein the reduced coenzyme Q10 preferably includes one or more selected from the group consisting of a reduced coenzyme Q10 Form I crystal, a reduced coenzyme Q10 Form II crystal, a co-crystal composed of reduced coenzyme Q10 and one or more other compounds, and an amorphous solid of reduced coenzyme Q10, more preferably includes one or more selected from the group consisting of a reduced coenzyme Q10 Form I crystal, a co-crystal composed of reduced coenzyme Q10 and one or more other compounds, and a reduced coenzyme Q10 Form II crystal.
(11) The method according to any one of (4) to (6), wherein the reduced coenzyme Q10 preferably includes one or more selected from the group consisting of a reduced coenzyme Q10 Form I crystal, a reduced coenzyme Q10 Form II crystal, a co-crystal composed of reduced coenzyme Q10 and one or more other compounds, and an amorphous solid of reduced coenzyme Q10, more preferably includes one or more selected from the group consisting of a reduced coenzyme Q10 Form I crystal, a co-crystal composed of reduced coenzyme Q10 and one or more other compounds, and a reduced coenzyme Q10 Form II crystal.
(12) The composition according to any one of (7) to (9), wherein the reduced coenzyme Q10 preferably includes one or more selected from the group consisting of a reduced coenzyme Q10 Form I crystal, a reduced coenzyme Q10 Form II crystal, a co-crystal composed of reduced coenzyme Q10 and one or more other compounds, and an amorphous solid of reduced coenzyme Q10, more preferably includes one or more selected from the group consisting of a reduced coenzyme Q10 Form I crystal, a co-crystal composed of reduced coenzyme Q10 and one or more other compounds, and a reduced coenzyme Q10 Form II crystal.

The present description encompasses the specifications and/or drawings in JP Patent Application Nos. 2021-210438 and 2022-152257 which serve as the basis of the priority of the present application.

### Advantageous Effects of Invention

According to the method for storing reduced coenzyme Q10 (QH) disclosed herein, oxidation of the QH can be controlled and the QH can be stably stored even when clathration or coating, or formulation such as capsulation, with other component, is not used to shield QH from oxygen.

According to the method for controlling oxidation of QH disclosed herein, oxidation of the QH can be effectively controlled.

According to the composition containing QH disclosed herein, oxidation of the QH can be controlled and the QH can be stably stored.

### Description of Embodiments

Hereinafter, the present invention will be described in detail.

### <Reduced Coenzyme Q10>

"Reduced coenzyme Q10" in the method according to one or more embodiments of the present invention may partially include oxidized coenzyme Q10, provided that reduced coenzyme Q10 is included as a main component. The "main component" herein means that it is included in a percentage of, for example, 50% by weight or more, generally 60% by weight or more, preferably 70% by weight or more, more preferably 80% by weight or more, further preferably 90% by weight or more, particularly preferably 95% by weight or more, and further particularly 98% by weight or more. Herein, the above-described percentages are the percentages of reduced coenzyme Q10 to the total weight of coenzyme Q10.

As mentioned above, reduced coenzyme Q10 has two forms of crystal polymorphisms, namely, Form I and Form II. Specifically, the crystal form of reduced coenzyme Q10 having a melting point around 48°C and showing characteristic peaks at diffraction angles (20 ± 0.2°) of 3.1°, 18.7°, 19.0°, 20.2°, and 23.0° in powder X-ray (Cu-Kα) diffraction is a Form I crystal, whereas the crystal form of reduced coenzyme Q10 having a melting point around 52°C and showing characteristic peaks at diffraction angles (2θ ± 0.2°) of 11.5°, 18.2°, 19.3°, 22.3°, 23.0°, and 33.3° in powder X-ray (Cu-Kα) diffraction is a Form II crystal.

In one or more embodiments of the present invention, the reduced coenzyme Q10 (QH) here used is preferably QH including one or more selected from the group consisting of a QH Form I crystal, a QH Form II crystal, a co-crystal composed of QH and one or more other compounds, and an amorphous solid of QH, particularly preferably QH including one or more selected from the group consisting of a QH Form I crystal, a co-crystal composed of QH and one or more other compounds, and a QH Form II crystal.

In the co-crystal composed of QH and one or more other compounds, such one or more other compounds included are not particularly limited as long as such each compound can form the co-crystal with QH, and examples thereof include organic carboxylic acids such as benzoic acid and derivatives thereof, organic alcohols including resorcinol, benzyl alcohol and phenol, and derivatives thereof, urea, and nicotinamide. Such one or more other compounds may be one compound, or two or more compounds, and are preferably one to three compounds.

The percentage of the QH Form I crystal in QH containing the Form I crystal is not particularly limited and is preferably, for example, 50% by weight or more, generally 60% by weight or more, preferably 70% by weight or more, more preferably 80% by weight or more, further preferably 90% by weight or more, particularly preferably 95% by weight or more, further particularly 98% by weight or more, and most preferably 100% by weight based on the total amount of QH. Examples of QH in a form other than the QH Form I crystal, which may be contained in QH containing a Form I crystal, may include one or more selected from the group consisting of a QH Form II crystal, a co-crystal composed of QH and one or more other compounds, and an amorphous solid of QH.

The percentage of the QH Form II crystal in QH containing the Form II crystal is not particularly limited and is preferably, for example, 50% by weight or more, generally 60% by weight or more, preferably 70% by weight or more, more preferably 80% by weight or more, further preferably 90% by weight or more, particularly preferably 95% by weight or more, further particularly 98% by weight or more, and most preferably 100% by weight based on the total amount of QH. Examples of QH in a form other than the QH Form II crystal, which may be contained in QH containing a Form II crystal, may include one or more selected from the group consisting of a QH Form I crystal, a co-crystal composed of QH and one or more other compounds, and an amorphous solid of QH.

The percentage of the co-crystal in QH containing the co-crystal composed of QH and one or more other compounds is not particularly limited and is preferably, for example, 50% by weight or more, generally 60% by weight or more, preferably 70% by weight or more, more preferably 80% by weight or more, further preferably 90% by weight or more, particularly preferably 95% by weight or more, further particularly 98% by weight or more, and most preferably 100% by weight based on the total amount of QH. Examples of QH in a form other than the co-crystal, which may be contained in QH containing the co-crystal, may include one or more selected from the group consisting of a QH Form I crystal, a QH Form II crystal, and an amorphous solid of QH.

QH used in one or more embodiments of the present invention is not required to be pre-formulated (i.e., need not be formulated in advance). It is preferable that QH in one or more embodiments of the present invention consists of non-preformulated QH, such as one or more selected from the group consisting of a QH Form I crystal, a QH Form II crystal, and a co-crystal composed of QH and one or more other compounds, because in such a case QH can be utilized in a wide range of applications. More preferably, QH is not pre-formulated QH (e.g., a clathrate of QH with cyclodextrin, QH dispersed in a matrix containing a water-soluble excipient in a particulate composition, QH coated with a coating material in a solid formulation, or a capsule of QH).

The water-soluble excipient can be, for example, one or more selected from the group consisting of a water-soluble polymer, a surfactant, sugar, and a yeast cell wall.

The coating material can be, for example, an oil-soluble coating material or a water-soluble coating material. The oil-soluble coating material can be, for example, a sugar ester of a higher fatty acid, shellac, a cellulose derivative, fatty acids and ester derivatives thereof, fats and oils, or zein. The water-soluble coating material can be, for example, gelatin, sugar, gum arabic, a sugar ester of a higher fatty acid, tragacanth, pectin, pullulan, alginic acid, dried albumen, milk, curdlan, a cellulose derivative, casein, a casein compound, starch, or a yeast cell wall.

The capsule is, for example, QH capsulated by a soft capsule, a hard capsule, a microcapsule, or the like. Examples of the material of the capsule may include gelatin derived from beef bone, bovine hide, pig hide, fishskin, or the like; a seaweed-derived product usable as a food additive, such as carrageenan or alginic acid; a plant seed-derived product such as locust bean gum or guar gum; an agent for production, containing cellulose; and starch such as wheat starch, potato starch, sweet potato starch, corn starch, or dextrin.

### <Method for Storing QH>

A first embodiment of the present invention relates to a method for storing QH, including storing a composition containing the QH and having a water activity at 25°C of 0.45 or less.

According to the method according to the present embodiment, oxidation of QH can be controlled and QH can be stably stored. A compound having low oxidation stability is generally considered to be more easily oxidized at a lower humidity because the lower the humidity, the higher the percentage of oxygen in air. The method according to the present embodiment utilizes the property that QH is stabilized in the state of being contained in a composition having a water activity at 25°C of 0.45 or less, which is contrary to the above common belief, to allow QH to be stably stored. Preparation of the composition is easily carried out. Therefore, the method according to the present embodiment can serve as a measure for efficiently stabilizing QH at a low cost.

QH in the form of a QH Form II crystal or a co-crystal composed of QH and one or more other compounds is itself costly to produce, and therefore a QH residual rate after storage (refer to the definition in Examples) of 85% or more is desired to provide QH in the above form at an appropriate price. The method according to the present embodiment is preferable in that the QH residual rate of QH containing at least one of a Form II crystal and a co-crystal composed of QH and one or more other compounds can be 85% or more.

QH in the form of a QH Form I crystal can be produced at a low cost, but is easily oxidized. Therefore, it is desired that the QH residual rate after storage (refer to the definition in Examples) is 40% or more, in order to provide QH in the above form at an appropriate price. The method according to the present embodiment is preferable in that the QH residual rate of QH containing a Form I crystal, after storage, can be 40% or more.

The composition containing QH and having a water activity at 25°C of 0.45 or less in the method according to the present embodiment will be described below.

The water activity of the composition can be measured by an ordinary method.

The water activity at 25°C of the composition may be 0.45 or less, and is preferably 0.43 or less, more preferably 0.42 or less, more preferably 0.40 or less, more preferably 0.38 or less, and particularly preferably 0.33 or less.

The composition may include one or more other components in addition to QH. The one or more other components may be any component usable in combination with QH, and examples thereof include a component acceptable as a food, a cosmetic product, or a pharmaceutical product.

The one or more other components is preferably a water-adsorbing substance, in order to control the water activity of the composition to 0.45 or less at 25°C. The water-adsorbing substance is a substance which adsorbs water vapor in a gas phase around the composition. Examples of the water-adsorbing substance include a component having a water activity at the temperature of an environment in which the composition is to be stored, of 0.45 or less, preferably 0.43 or less, more preferably 0.42 or less, more preferably 0.40 or less, more preferably 0.38 or less, and particularly preferably 0.33 or less. Examples of the water-adsorbing substance usable as the one or more other components may include cellulose, starch, sugar, other water-soluble polymer compound, and silicon dioxide, and the water-adsorbing substance is preferably one or more selected from the group consisting of cellulose, starch, sugar, and other water-soluble polymer compound.

Examples of the cellulose may include crystalline cellulose, a cellulose powder, methylcellulose, ethylcellulose, hydroxymethylcellulose, hydroxypropylcellulose, hydroxypropylmethylcellulose, carboxymethylcellulose and any salt thereof.

Examples of the starch may include wheat starch, potato starch, sweet potato starch, corn starch, dextrin, hydroxypropyl starch, starch acetate, oxidized starch, starch octenylsuccinate and any salt thereof, and partially pregelatinized starch.

The dextrin is not particularly limited as long as it is a degraded product of starch, and any low molecular dextrin or high molecular dextrin can be suitably used. Maltodextrin, cyclodextrin, cluster dextrin, or the like can also be suitably used.

Examples of the sugar may include monosaccharides such as glucose, fructose, galactose, arabinose, xylose and mannose; disaccharides such as maltose, sucrose, lactose and trehalose; oligosaccharides such as fructo-oligosaccharide, soybean oligosaccharide, galacto-oligosaccharide and xylo-oligosaccharide; and sugar alcohols such as sorbitol, maltitol, erythritol, lactitol, and xylitol.

Examples of such other water-soluble polymer compound may include gum arabic, gelatin, agar, tragacanth, pectin, carrageenan, casein, a casein compound, dried albumen, milk, curdlan, alginic acids, soybean polysaccharides, pullulan, xanthan gum, and locust bean gum.

In a preferred aspect, the composition is a mixed composition in which QH and one or more other components are mixed. The mixed composition can be a composition acceptable as a food, a cosmetic product, or a pharmaceutical product, as a whole.

The mixed composition may be a mixed composition in which QH and one or more other components are homogeneously mixed, or may be a mixed composition in which QH and one or more other components are heterogeneously mixed. Such a mixed composition homogeneously mixed refers to a composition containing QH and one or more other components, in which the concentration distribution of QH in the entire composition is homogeneous or substantially homogeneous. Such a mixed composition homogeneously mixed can be obtained by, for example, sufficiently mixing QH and one or more other components. Such a mixed composition heterogeneously mixed refers to a composition containing QH and one or more other components, in which the concentration distribution of QH is not homogeneous and is biased. Such a mixed composition heterogeneously mixed can be obtained by, for example, adding QH to one or more other components such as a material for foods.

In another preferred aspect, the composition is a multiphase composition including a first phase containing QH and a second phase containing one or more other components, in which the phases are in contact with each other. The multiphase composition can be a composition acceptable as a food, a cosmetic product, or a pharmaceutical product, as a whole, or at least the first phase can be a composition acceptable as a food, a cosmetic product, or a pharmaceutical product when the first phase and the second phase can be separated. The first phase refers to a phase composed of QH or a homogeneous phase containing QH. The second phase refers to a phase which can be in contact with the first phase without being mixed with the first phase. The multiphase composition can be, for example, one in which the first phase and the second phase are stacked, or one in which one of the first phase and the second phase is supported on the other. One example of the multiphase composition is one including a first phase composed of QH-containing particles and a second phase composed of one or more other components in matrix form, in which the first phase is supported on the second phase. Another example of the multiphase composition is one including a first phase containing QH and a second phase containing a water-adsorbing substance, in which the first phase and the second phase are in contact with each other.

The content of QH in the composition is not particularly limited. The content of QH based on the total mass of the composition can be, for example, 0.01% by mass or more, preferably 0.1% by mass or more, and more preferably 1% by mass or more, and can be, for example, 99% by mass or less, preferably 90% by mass or less, and more preferably 50% by mass or less.

The temperature at which the composition is stored in the method according to the present embodiment is, for example, a temperature of -25°C or more and 50°C or less, preferably a temperature of -20°C or more, -10°C or more, 0°C or more, 4°C or more, 10°C or more, 15°C or more, 20°C or more, or 25°C or more, and preferably a temperature of 45°C or less, or 40°C or less. The temperature can be specifically 25°C or 40°C.

The period for which the composition is stored in the method according to the present embodiment is not particularly limited as long as it is a period until use of a product after production, and can be appropriately adjusted depending on storage conditions such as temperature. The period is preferably 3 days or more, 1 week or more or 2 weeks or more and can be, for example, 5 years or less, generally 3 years or less, preferably 2 years or less, more preferably 1 year or less, further preferably 6 months or less, further preferably 8 weeks or less, and most preferably 6 weeks or less, 5 weeks or less, or 4 weeks or less.

When the composition is stored in the method according to the present embodiment, the composition is preferably stored as a packaged body accommodated in a container and tightly sealed in order to prevent volatilization of water. The packaged body may include a gas phase in the container, and the gas phase may be air. A packaged body including air as the gas phase is preferable because it can be produced at a low cost compared to a packaged body including an inert gas such as nitrogen as the gas phase.

### <Method for Controlling Oxidation of QH>

A second embodiment of the present invention relates to a method for controlling oxidation of QH, including storing a composition containing QH and having a water activity at 25°C of 0.45 or less.

According to the method according to the present embodiment, oxidation of QH can be efficiently controlled at a low cost.

Respective characteristics and preferred aspects of QH, the composition, and the step of storing the composition in the method according to the present embodiment are as described with respect to the method according to the first embodiment.

### <Composition>

A third embodiment of the present invention relates to a composition containing QH and having a water activity at 25°C of 0.45 or less.

Oxidation of QH in the form of the composition of the present embodiment is effectively controlled.

Respective characteristics and preferred aspects of QH and the composition in the composition according to the present embodiment are as described with respect to the method according to the first embodiment.

### Examples

### 1. Raw Materials

The present invention will be further specifically described with reference to the following Examples, but the present invention is not limited to these Examples. In Examples, reduced coenzyme Q10 (trade name: Kaneka QH) manufactured by KANEKA CORPORATION was used as a reduced coenzyme Q10 Form I crystal (QH Form I crystal).

### 2. Method for Evaluating Oxidation Stability

The weight ratio of reduced coenzyme Q10 to total coenzyme Q10 (namely, reduced coenzyme Q10/(oxidized coenzyme Q10 + reduced coenzyme Q10)) is defined as "QH ratio". The QH ratio was determined by the following HPLC analysis. For the evaluation of oxidation stability, a rate of the change in the QH ratio at the end of the evaluation from that at the beginning of the evaluation was defined as "QH residual rate", and the QH residual rate determined from the following expression was used as a measure of oxidation stability. QH residual rate (%) = 100 × QH ratio at the end of the evaluation/QH ratio at the beginning of the evaluation

### (HPLC Analysis Conditions)

Column: SYMMETRY C18 (manufactured by Waters); 250 mm (length), 4.6 mm (inner diameter)
Mobile phase: C₂H₅OH: CH₃OH = 4:3 (v: v)
Detection wavelength: 210 nm
Flow rate: 1 mL/min

### 3. Method for Measuring Water Activity

The water activity was measured with AquaLab Series 4TE (METER Group Inc.). A sample having a volume of about 5 ml in a sample dish was placed in AquaLab Series 4TE, and the water activity at 25°C was measured.

### 4. Method for Preparing Reduced Coenzyme Q10 Form II Crystal (QH Form II Crystal)

To 611 g of ethanol, 89 g of the QH Form I crystal was added, and heated to 50°C to completely dissolve the QH Form I crystal. This solution was cooled, and 1.8 g of a reduced coenzyme Q10 Form II crystal prepared according to the description of Patent Literature 2 was added as a seed crystal when the temperature of the solution reached 36°C. The solution was cooled to 33.5°C over 7 hours, thereafter cooled to 25°C at a rate of 1°C/hour, and further cooled to 1°C at a rate of 10°C/hour to obtain a white slurry. The slurry obtained was filtered under reduced pressure to obtain a wet crystal, and the wet crystal was washed with cold ethanol and further dried under reduced pressure to obtain a QH Form II crystal.

### 5. Storage of Reduced Coenzyme Q10 in Composition Having Controlled Water Activity

### [Example 1]

The QH Form II crystal and each excipient shown in Table 1 were mixed at a weight ratio of 1:1 to provide a QH Form II crystal-containing composition. In a glass vial (volume 33 ml), 0.2 g of the composition was placed, and the vial was sealed. This packaged body was stored under a temperature of 40°C and a relative humidity of 75% for 4 weeks, after which the QH residual rate was determined. The water activity at 25°C of the composition was measured, and shown, together with the QH residual rate, in Table 1.

**[Table 1]**

| | Excipient | Water activity | QH residual rate (%) |
|---|---|---|---|
| Example 1-1 | Carboxymethylcellulose | 0.27 | 94.1 |
| Example 1-2 | Partially pregelatinized starch | 0.31 | 92.0 |
| Example 1-3 | Microcrystalline cellulose | 0.33 | 93.7 |
| Example 1-4 | Maltodextrin | 0.42 | 90.4 |

### [Example 2]

The QH Form II crystal and gum arabic dried with a vacuum dryer were mixed at a weight ratio of 1:1 to provide a QH Form II crystal-containing composition. In a glass vial (volume 10 ml), 0.03 g of the composition was placed, and the vial was sealed. This packaged body was stored for 4 weeks under a temperature of 40°C and a relative humidity of 75% or under a temperature of 25°C and a relative humidity of 60%, after which the QH residual rate was determined. The water activity at 25°C of the composition was measured, and shown, together with the QH residual rate, in Table 2.

**[Table 2]**

| | Excipient | Storage temperature (°C) | Water activity | QH residual rate (%) |
|---|---|---|---|---|
| Example 2-1 | Gum arabic | 40 | 0.04 | 93.2 |
| Example 2-2 | Gum arabic | 25 | 0.04 | 96.8 |

### [Comparative Example 1]

In a container having a controlled internal relative humidity of 48%, 0.2 g of the QH Form II crystal was enclosed, and the container was sealed. The water activity of the QH Form II crystal in this packaged body was 0.48. The packaged body was stored under a temperature of 40°C and a relative humidity of 75% for 4 weeks, after which the QH residual rate was determined and shown in Table 3.

**[Table 3]**

| | Water activity | QH residual rate (%) |
|---|---|---|
| Comparative Example 1 | 0.48 | 82.1 |

### Tables 1, 2, and 3 show that, when an excipient was added to a QH Form II crystal such that the composition had a water activity of 0.45 or less, the QH Form II crystal in the composition was stably stored.

### [Example 3]

The QH Form I crystal and carboxymethylcellulose were mixed at a weight ratio of 1:1 to provide a QH Form I crystal-containing composition. In a glass vial (volume 33 ml), 0.2 g of the composition was placed, and the vial was sealed. This packaged body was stored under a temperature of 40°C and a relative humidity of 75% for 2 weeks, after which the QH residual rate was determined. The water activity at 25°C of the composition was measured, and shown, together with the QH residual rate, in Table 4.

**[Table 4]**

| | Excipient | Water activity | QH residual rate (%) |
|---|---|---|---|
| Example 3 | Carboxymethylcellulose | 0.28 | 48.8 |

### [Example 4]

The QH Form I crystal and gum arabic dried with a vacuum dryer were mixed at a weight ratio of 1:1 to provide a QH Form I crystal-containing composition. In a glass vial (volume 10 ml), 0.03 g of the composition was placed, and the vial was sealed. This packaged body was stored under a temperature of 25°C and a relative humidity of 60% for 4 weeks, after which the QH residual rate was determined. The water activity at 25°C of the composition was measured, and shown, together with the QH residual rate, in Table 5.

**[Table 5]**

| | Excipient | Water activity | QH residual rate (%) |
|---|---|---|---|
| Example 4 | Gum arabic | 0.05 | 45.2 |

### [Comparative Example 2]

In a container having a controlled internal relative humidity of 48%, 0.2 g of the QH Form I crystal was enclosed, and the container was sealed. The water activity of the QH Form I crystal in this packaged body was 0.48. The packaged body was stored under a temperature of 40°C and a relative humidity of 75% for 2 weeks, after which the QH residual rate was determined and shown in Table 6.

**[Table 6]**

| | Water activity | QH residual rate (%) |
|---|---|---|
| Comparative Example 2 | 0.48 | 37.2 |

The results shown in Tables 4, 5, and 6 revealed that, when an excipient was added to a QH Form I crystal such that the composition had a water activity of 0.45 or less, the QH Form I crystal in the composition was stably stored.

### 6. Method for Producing Co-crystal Composed of Reduced Coenzyme Q10 and Nicotinamide

To 7.85 g of ethanol, 4.33 g of the QH Form I crystal and 1.22 g of nicotinamide were added, and heated to 50°C, thereby completely dissolving the QH Form I crystal and nicotinamide. This solution was cooled, and the resulting white slurry was dried under reduced pressure, thereby obtaining a co-crystal composed of QH and nicotinamide. The melting point of the co-crystal obtained was measured with a differential scanning calorimeter (DSC200, manufactured by Hitachi, Ltd., heating rate: 1°C/min), and a peak was observed at a temperature different from the melting point (48°C) of the QH Form I crystal and the melting point (127°C) of nicotinamide, which were used as raw materials.

### [Example 5]

In a packaged body having a controlled relative humidity of 11%, 0.2 g of a co-crystal composed of QH and nicotinamide was enclosed in an opened state. The water activity of the co-crystal in the packaged body was here 0.11. The packaged body was stored at 40°C for 2 weeks, after which the QH residual rate was determined.

### [Comparative Example 3]

In a packaged body having a controlled internal relative humidity of 48%, 0.2 g of a co-crystal composed of QH and nicotinamide was enclosed in an opened state. The water activity of the co-crystal in the packaged body was here 0.48. The packaged body was stored at 40°C for 2 weeks, after which the QH residual rate was determined.

### The results obtained in Example 10 and Comparative Example 3 are summarized and shown in Table 7.

**[Table 7]**

| | Storage temperature (°C) | QH residual rate (%) |
|---|---|---|
| Example 5 | 40 | 94.7 |
| Comparative Example 3 | 40 | 82.6 |

Table 7 shows that QH present in a co-crystal composed of QH and nicotinamide is also stably stored in a composition having a water activity of 0.45 or less.

All publications, patents, and patent applications cited in the present description are incorporated herein by reference in their entirety.

The upper and/or lower limits of the numerical ranges described herein can be combined arbitrarily to define a preferred range. For example, a preferred range can be defined by arbitrarily combining the upper and lower limits of the numerical ranges, a preferred range can be defined by arbitrarily combining the upper limits of the numerical ranges, and a preferred range can be defined by arbitrarily combining the lower limits of the numerical ranges. The numerical range represented with the term "to" in the present application includes respective numerical values described before and after the term "to" as the upper and lower limits thereof.

It should be understood that throughout the present description, expressions in the singular forms include the concept of their plural forms unless otherwise specifically stated. Thus, articles in the singular forms (e.g., "a," "an," and "the" in the English language) should be understood to include the concept of their plural forms, unless otherwise specifically stated.

Although the embodiment of the present invention has been described in detail above, the specific configuration is not limited to this embodiment, and even when there are design changes within the scope of the present disclosure, they are included in the present disclosure.

## Claims

1. A method for storing reduced coenzyme Q10, comprising
storing a composition containing reduced coenzyme Q10 and having a water activity at 25°C of 0.45 or less.

2. The method according to claim 1, wherein the composition is a mixed composition in which the reduced coenzyme Q10 and one or more other components are mixed.

3. The method according to claim 1, wherein the composition is a multiphase composition including a first phase containing the reduced coenzyme Q10 and a second phase containing one or more other components, the first and second phases being in contact with each other.

4. A method for controlling oxidation of reduced coenzyme Q10, comprising
storing a composition containing reduced coenzyme Q10 and having a water activity at 25°C of 0.45 or less.

5. The method according to claim 4, wherein the composition is a mixed composition in which the reduced coenzyme Q10 and one or more other components are mixed.

6. The method according to claim 4, wherein the composition is a multiphase composition including a first phase containing the reduced coenzyme Q10 and a second phase containing one or more other components, the first and second phases being in contact with each other.

7. A composition containing reduced coenzyme Q10 and having a water activity at 25°C of 0.45 or less.

8. The composition according to claim 7, wherein the composition is a mixed composition in which the reduced coenzyme Q10 and one or more other components are mixed.

9. The composition according to claim 7, wherein the composition is a multiphase composition including a first phase containing the reduced coenzyme Q10 and a second phase containing one or more other components, the first and second phases being in contact with each other.
